# EUROPÄISCHE PATENTANMELDUNG

(11) **EP 2 103 273 A1**
(43) Veröffentlichungstag der Anmeldung: **23.09.2009**
(21) Anmeldenummer: 09152634.3
(22) Anmeldetag: 12.02.2009
(51) Int. Cl.: A61C 1/14, A61C 3/03

(54) **Befestigungssystem**

(30) Priorität: 18.03.2008 DE 102008014667
(71) Anmelder: Ferton Holding SA, 2800 Delemont (CH)
(72) Erfinder: Wurm, Helmut, 1716, Schwarzsee (CH); Heini, Dominique, 1162, St-Prex (CH); Gras, Guillaume, 1110, Morges (CH); Lehner, Frédéric, 1241, Puplinge (CH)
(74) Vertreter: Bauer, Clemens

(57) **Zusammenfassung**

Befestigungssystem zur Befestigung eines medizinischen Instruments an einer Aufnahmevorrichtung, wobei das medizinische Instrument derart mit der Aufnahmevorrichtung koppelbar ist, dass ein sich verjüngender Teil der Aufnahmevorrichtung oder des medizinischen Instruments mit einem im Wesentlichen formkongruenten Teil des verbleibenden medizinischen Instruments oder der Aufnahmevorrichtung in Eingriff bringbar ist.

## Beschreibung

Die vorliegende Erfindung bezieht sich auf ein Befestigungssystem zur Befestigung eines medizinischen Instruments an einer Aufnahmevorrichtung, auf ein medizinisches Instrument, insbesondere zahnärztliches Instrument zur Beseitigung von Zahnstein oder zur Behandlung von Parodontose, sowie auf eine Aufnahmevorrichtung für ein medizinisches Instrument, insbesondere eines Handstücks für ein zahnärztliches Instrument zur Beseitigung von Zahnstein oder zur Behandlung von Parodontose.

Befestigungssysteme der in Frage stehenden Art sind hinlänglich aus dem Stand der Technik bekannt. Diese dienen insbesondere der Verbindung zwischen einem Handstück und einem sog. Scaler, d.h. einem Instrument zur Beseitigung von Zahnstein oder zur Behandlung von Parodontose. Bisherige System sind insbesondere derart gestaltet, dass ein Endabschnitt des medizinischen Instruments ein Gewinde aufweist, welches auf eine entsprechende Aufnahmevorrichtung soweit aufgeschraubt wird, bis die Stirnfläche der Seite des medizinischen Instruments, an welcher das Gewinde ausgebildet ist, mit einer entsprechenden Anschlagfläche der Aufnahmevorrichtung in Kontakt tritt. Problematisch bei diesen Systemen ist jedoch, dass die Verbindung der zwei Elemente oftmals nicht zuverlässig erfolgt, d.h. insbesondere sich die beiden Elemente während der Nutzung voneinander lösen. Dies ist insbesondere Fall, wenn das medizinische Instrument in Ultraschallschwingungen versetzt wird. Sollten die Elemente einen Kanal zur Durchleitung eines Fluids aufweisen, so neigen die bekannten Systeme oftmals zu Leckagen, da die Verbindungsstelle keine ausreichende Dichtigkeit aufweist.

Es ist somit Aufgabe der vorliegenden Erfindung, ein Befestigungssystem zur Befestigung eines medizinischen Instruments an einer Aufnahmevorrichtung, ein medizinisches Instrument, insbesondere zahnärztliches Instrument zur Beseitigung von Zahnstein oder zur Behandlung von Parodontose, sowie eine Aufnahmevorrichtung für ein medizinisches Instrument, insbesondere ein Handstück für ein zahnärztliches Instrument zur Beseitigung von Zahnstein oder zur Behandlung von Parodontose, vorzusehen, welche eine zuverlässige Befestigung des medizinischen Instruments an der Aufnahmevorrichtung gewährleisten.

Diese Aufgabe wird durch ein Befestigungssystem zur Befestigung eines medizinischen Instruments an einer Aufnahmevorrichtung mit den Merkmalen des Anspruchs 1, ein medizinisches Instrument, insbesondere zahnärztliches Instrument zur Beseitigung von Zahnstein oder zur Behandlung von Parodontose, mit den Merkmalen des Anspruchs 15, sowie durch eine Aufnahmevorrichtung für ein medizinisches Instrument, insbesondere ein Handstück für ein zahnärztliches Instrument zur Beseitigung von Zahnstein oder zur Behandlung von Parodontose, mit den Merkmalen des Anspruchs 24, gelöst. Bevorzugte Ausführungsformen ergeben sich aus den abhängigen Ansprüchen.

Erfindungsgemäß ist ein Befestigungssystem zur Befestigung eines medizinischen Instruments an einer Aufnahmevorrichtung vorgesehen, wobei das medizinische Instrument derart mit der Aufnahmevorrichtung koppelbar ist, dass ein sich verjüngender Teil der Aufnahmevorrichtung oder des medizinischen Instruments mit einem im Wesentlichen formkongruenten Teil des verbleibenden medizinischen Instruments oder der Aufnahmevorrichtung in Eingriff bringbar ist. Es wird somit ein Befestigungssystem bereitgestellt, mittels welchem ein medizinisches Instrument, welches insbesondere zur Zahnbehandlung dient, an einer vorteilhafterweise als Handstück ausgebildeten Aufnahmevorrichtung befestigbar ist. Das medizinische Instrument kann insbesondere als so genannter Scaler ausgebildet sein, der während des Betriebs vorzugsweise in Schwingungen, vorzugsweise in Ultraschallschwingungen, versetzt wird. Dies erfolgt mittelbar oder unmittelbar über die Aufnahmevorrichtung, die als Teil eines Handstücks, welches vom Benutzer während der Behandlung gehalten wird, ausgebildet sein kann. Es versteht sich, dass das medizinische Instrument auch beispielsweise ein Ultraschall-Lithotriptor oder sonstiges beliebiges orthopädisches Werkzeug sein kann. An der Aufnahmevorrichtung oder dem medizinischen Instrument ist vorteilhafterweise ein sich verjüngender Teil bzw. Bereich bzw. Abschnitt ausgebildet, der mit einem im Wesentlichen formkongruenten Teil bzw. Bereich bzw. Abschnitt des verbleibenden Elements, d.h. des medizinischen Instruments oder der Aufnahmevorrichtung, in Eingriff bringbar ist. Der sich verjüngende Teil ist hierbei derart ausgebildet, dass dieser sich im Wesentlichen in Richtung eines distalen Endes des entsprechenden Elements (d.h. des medizinischen Instruments oder der Aufnahmevorrichtung) hin verjüngt. Insbesondere bildet der sich verjüngende Teil das distale Ende des entsprechenden Elements aus. Der sich verjüngende Teil kann vorteilhafterweise im Wesentlichen rotationssymetrisch um eine Längsachse des Elements ausgebildet sein. In anderen Worten kann der sich verjüngende Teil im Wesentlichen konzentrisch um die Längsachse angeordnet sein. Das medizinische Instrument kann insbesondere einteilig, bzw. einstückig, bzw. als ein einziges Bauteil ausgebildet sein. Entsprechendes gilt für die Aufnahmevorrichtung. Durch den sich verjüngenden Teil bzw. Bereich kontaktieren entsprechende Abschnitte des medizinischen Instruments und der Aufnahmeeinrichtung derart miteinander, dass dieser Kontaktbereich gegenüber einem Fluid dicht ist. Aufgrund der Reibung zwischen den sich kontaktierenden Flächen bzw. Bereichen wird gewährleistet, dass die Elemente sicher miteinander verbunden sind und auch während des Betriebs sich nicht voneinander lösen können. Schließlich wird durch das erfindungsgemäße Befestigungssystem erreicht, dass sich die Elemente zueinander aufgrund des Eingriffs des sich verjüngenden Teils mit einem entsprechenden, im Wesentlichen formkongruenten Teil zueinander zentrieren. Der Begriff "im Wesentlichen formkongruent" ist hierbei insoweit zu verstehen, dass zumindest ein Teil der äußeren Fläche eines Elements einem Teil der inneren Fläche des verbleibenden Elements entspricht, so dass die Elemente (d.h. das medizinische Instrument und die Aufnahmevorrichtung) in diesem Bereich zusammenpassen. Hierfür sind vorteilhafterweise ein Kontaktbereich an dem medizinischen Instrument und ein hiermit in Kontakt bringbarer Kontaktabschnitt an der Aufnahmevorrichtung vorgesehen. Im aneinander befestigten Zustand kontaktieren der Kontaktbereich und der Kontaktabschnitt derart, dass diese vorzugsweise in einem Flächenkontakt zueinander stehen.

Zweckmäßigerweise ist der sich verjüngende Teil an dem Kontaktbereich oder dem Kontaktabschnitt ausgebildet. Somit wird vorteilhafterweise erreicht, dass sich das medizinische Instrument und die Aufnahmevorrichtung zumindest in dem Bereich des sich verjüngenden Teils und dem entsprechenden gegenüberliegenden Bereich des anderen Elements kontaktieren, so dass die Dichtungswirkung insbesondere durch diesen Bereich erzielt wird. Der Kontaktbereich und/oder der Kontaktabschnitt, zumindest deren sich kontaktierende Flächen, sind zweckmäßigerweise im Wesentlichen rotationssymetrisch um die Längsachse des jeweiligen Elements ausgebildet.

Vorteilhafterweise besitzt der sich verjüngende Teil eine konusförmige Konfiguration. Der verjüngende Teil kann somit eine kegelstumpfförmige Konfiguration besitzen, wobei die Mantelfläche des sich verjüngenden Teils in einer parallel zur Längsachse des Elements liegenden Querschnittsfläche gerade, als eine Aneinanderreihung einer Vielzahl von Geraden oder gekrümmt, vorzugsweise rund, ausgebildet sein kann. Durch die kegelstumpfförmige Konfiguration wird vorteilhafterweise erreicht, dass sich die beiden Elemente zueinander während der Montage selbst zentrieren. Hierbei kann die Konusauflagefläche hinsichtlich ihrer Größe, aber auch ihrer Neigung zur Längsachse der Elemente in Abhängigkeit vom Einsatzzweck ausgebildet sein. Je größer der Verjüngungswinkel (d.h. Winkel der Mantelfläche zur Längsachse des sich verjüngenden Teils) bzw. der Konuswinkel bzw. der Kegelwinkel ist, umso steifer ist die Befestigung des medizinischen Instruments an der Aufnahmevorrichtung. Infolgedessen lässt sich die Befestigung quasi einstellen. Als besonders zweckmäßig haben sich hier Winkel zwischen etwa 2 bis 35°, vorzugsweise etwa 3 bis 25° und besonders bevorzugt etwa 4 bis 10° erwiesen. In einem im Wesentlichen quer- bzw. senkrecht bzw. rechtwinklig zur Längsachse des sich verjüngenden Teils gesehenen Querschnitt kann dieser eine beliebige Konfiguration aufweisen, vorzugsweise eckig, gekrümmt und besonders zweckmäßigerweise rund.

Weiterhin bevorzugt sind das medizinische Instrument und die Aufnahmevorrichtung als Hohlkörper zur Durchführung eines Fluids ausgebildet. Die Elemente (d.h. medizinisches Instrument und Aufnahmevorrichtung) weisen somit einen Durchlass bzw. einen Kanal auf, so dass diese im Wesentlichen als Rohr bzw. rohrförmig ausgebildet sind. Besonders vorteilhafterweise sind daher der Kontaktbereich und der Befestigungsbereich an einer innen liegenden bzw. inneren Mantelfläche der als Hohlkörper ausgebildeten Elemente ausgebildet.

In einer bevorzugten Ausführungsform sind ein Befestigungsbereich an dem medizinischen Instrument und ein hiermit in Eingriff bringbarer Befestigungsabschnitt an der Aufnahmevorrichtung vorgesehen. Befestigungsbereich und Befestigungsabschnitt sind hierbei vorzugsweise im Wesentlichen rotationssymetrisch um die Längsachse des jeweiligen Elements ausgebildet, wobei Abweichungen von der Rotationssymetrie aufgrund der Ausbildung eines Gewindes in diesem Bereich zu vernachlässigen sind.

Vorteilhafterweise entspricht die Längsachse bzw. Rotationssymetrielinie des Kontaktbereichs im Wesentlichen der Längsachse bzw. Rotationssymetrielinie des Befestigungsbereichs. Entsprechendes gilt ebenfalls für den Kontaktabschnitt und Befestigungsabschnitt der Aufnahmevorrichtung.

Zweckmäßigerweise weisen der Befestigungsbereich und der Befestigungsabschnitt jeweils Eingriffsmittel zum gegenseitigen Eingriff auf. Die vorteilhafterweise lösbare Befestigung des medizinischen Instruments an der Aufnahmevorrichtung erfolgt somit durch einen Eingriff der jeweiligen Eingriffsmittel. Die Funktion der Dichtung wird hingegen von der Kontaktierung des sich verjüngenden Teils mit dem entsprechend im Wesentlichen formkongruenten Teil des anderen Elements bereitgestellt.

Vorteilhafterweise sind die Eingriffsmittel des Befestigungsabschnitts außen liegend angeordnet. In anderen Worten können die Eingriffsmittel des Befestigungsabschnitts an einer äußeren Mantelfläche der Aufnahmevorrichtung ausgebildet sein.

Entsprechend können die Eingriffsmittel des Befestigungsbereichs an der innen liegenden Mantelfläche des als Hohlkörper ausgebildeten medizinischen Instruments ausgebildet sein.

Vorzugsweise sind die Eingriffsmittel als Schraubgewinde in Form eines Trapezgewindes ausgebildet. Dies ermöglicht eine besonders große Auflagefläche zwischen den Elementen in deren Eingriffsbereich, so dass bei einer Anregung des Instruments entsprechende Schwingungen besonders optimal übertragen werden können. Darüber hinaus können durch ein Trapezgewinde im Vergleich zu einem herkömmlichen Spitzgewinde wesentlich höhere Kräfte übertragen werden.

Bevorzugterweise weist das Schraubgewinde weniger als sechs, vorzugsweise weniger als vier und besonders bevorzugterweise zwei Gewindegänge auf. Durch ein derartiges Kurzgewinde wird die Operabilität bzw. Bedienbarkeit des Befestigungssystems weiter verbessert.

In einer weiteren bevorzugten Ausführungsform weist das medizinische Instrument zwischen Befestigungsbereich und Kontaktbereich einen Deformationsbereich auf. Der Deformationsbereich dient insbesondere einer Dehnung des medizinischen Instruments in diesem Bereich bei der Montage an der Aufnahmevorrichtung, wobei aufgrund der Schwingungsanregung, beispielsweise durch Ultraschall, des medizinischen Instruments eine weiterführende, überlagerte und wechselnde Dehnung zusätzlich erfolgt. Die Summe aller Dehnungen, resultierend aus der Vorspannung und der schwingungsbedingten Dehnung, sollte dabei aber vorzugsweise zu jedem Zeitpunkt der Schwingungsperiode größer als Null sein. Hierdurch wird vorteilhafterweise erreicht, dass eine Phasengleichheit der Schwingungen des sich verjüngenden Teils und des Befestigungsabschnitts bzw. Befestigungsbereichs vorliegt, so dass keine Relativbewegung zwischen Aufnahmevorrichtung und medizinischem Instrument möglich ist. Dies führt zu einer besonders vorteilhaften Dichtung der beiden Elemente gegeneinander und zu einer besonders zuverlässigen Befestigung bzw. Verbindung.

Entsprechend kann die Aufnahmevorrichtung zwischen Befestigungsabschnitt und Kontaktabschnitt einen Deformationsabschnitt aufweisen.

Zweckmäßigerweise ist das Produkt aus Querschnittsfläche des Deformationsbereichs und Elastizitätsmodul des Materials des medizinischen Instruments im Wesentlichen gleich dem Produkt aus Querschnittsfläche des Deformationsabschnitts und Elastizitätsmodul des Materials der Aufnahmevorrichtung. Das Produkt aus Querschnittsfläche des Deformationsbereichs und Elastizitätsmodul des Materials des medizinischen Instruments kann hierbei vorteilhafterweise zwischen etwa 0,7 bis etwa 1,4, vorzugsweise etwa 0,85 bis etwa 1,2 und besonders vorteilhafterweise etwa 0,95 bis etwa 1,05 mal dem Produkt aus Querschnittsfläche des Deformationsbereichs und Elastizitätsmodul des Materials der Aufnahmevorrichtung sein. Hierdurch wird - unabhängig von der Wahl des Materials der Elemente - eine besonders zuverlässige Befestigung und Dichtigkeit der Elemente gegeneinander gewährleistet.

Weiterhin erfindungsgemäß ist ein medizinisches Instrument, insbesondere zahnärztliches Instrument zur Beseitigung von Zahnstein oder zur Behandlung von Parodontose, vorgesehen, umfassend einen Behandlungsbereich und einen Montagebereich zur Befestigung an einer Aufnahmevorrichtung, wobei der Montagebereich einen sich verjüngenden Teil aufweist, um mit einem im Wesentlichen formkongruenten Teil der Aufnahmevorrichtung in Eingriff gebracht zu werden. Das medizinische Instrument kann zweckmäßigerweise ein so genannter Scaler sein, der zur Beseitigung von Zahnstein oder zur Behandlung von Parodontose, insbesondere zur Beseitigung von Zahnstein, dient. Hierfür weist das medizinische Instrument einen Behandlungsbereich auf, der insbesondere an einem distalen Ende bzw. Endbereich vorgesehen ist und über welchen die Behandlung beim Patienten erfolgt. An einem diesem im Wesentlichen gegenüberliegenden Bereich weist das medizinische Instrument einen Montagebereich auf, um dieses an einer entsprechenden Aufnahmevorrichtung eines Teils eines Handstücks zu befestigen.

Zweckmäßigerweise ist ein Kontaktbereich an dem medizinischen Instrument vorgesehen, an welchem der sich verjüngende Teil ausgebildet ist.

Vorteilhafterweise besitzt der sich verjüngende Teil eine konusförmige Konfiguration.

Weiterhin vorteilhafterweise ist das medizinische Instrument als Hohlkörper zur Durchführung eines Fluids ausgebildet.

Zweckmäßigerweise ist ein Befestigungsbereich mit Eingriffsmitteln zur Schaffung eines Eingriffs mit der Aufnahmevorrichtung vorgesehen. Bevorzugterweise sind die Eingriffsmittel des Befestigungsbereichs an einer innen liegenden Mantelfläche des als Hohlkörper ausgebildeten medizinischen Instruments ausgebildet, wobei diese ferner zweckmäßigerweise als Schraubgewinde in Form eines Trapezgewindes ausgebildet sind. Hierbei kann das Schraubgewinde weniger als sechs, vorzugsweise weniger als vier und besonders bevorzugterweise zwei Gewindegänge aufweisen.

In einer weiteren bevorzugten Ausführungsform ist zwischen Befestigungsbereich und Kontaktbereich ein Deformationsbereich vorgesehen. Es versteht sich, dass die weiteren Vorteile und Merkmale des erfindungsgemäßen Befestigungssystems ebenfalls in dem erfindungsgemäßen medizinischen Instrument Anwendung finden können.

Weiterhin erfindungsgemäß ist eine Aufnahmevorrichtung für ein medizinisches Instrument, insbesondere ein Handstück für ein zahnärztliches Instrument zur Beseitigung von Zahnstein oder zur Behandlung von Parodontose, vorgesehen, umfassend einen Montageabschnitt mit einem sich verjüngenden Teil, um mit einem im Wesentlichen formkongruenten Teil des medizinischen Instruments in Eingriff gebracht zu werden.

Es versteht sich, dass die weiteren Vorteile und Merkmale des erfindungsgemäßen Befestigungssystems sowie des erfindungsgemäßen medizinischen Instruments ebenfalls in der erfindungsgemäßen Aufnahmevorrichtung Anwendung finden können.

Weitere Vorteile und Merkmale der Erfindung ergeben sich aus der nachfolgenden Beschreibung von bevorzugten Ausführungsformen der Erfindung, wobei einzelne Merkmale und Aspekte verschiedener Ausführungsformen zu neuen Ausführungsformen miteinander kombinierbar sind. Es zeigt:
- Fig. 1: eine perspektivische Ansicht einer bevorzugten Ausführungsform des erfindungsgemäßen medizinischen Instruments;
- Fig. 2: eine Querschnittsansicht eines Teils einer bevorzugten Ausführungsform des erfindungsgemäßen Instruments;
- Fig. 3: eine Querschnittsansicht einer bevorzugten Ausführungsform der erfindungsgemäßen Aufnahmevorrichtung.

Das Befestigungssystem in einer bevorzugten, beispielhaften Ausführungsform dient der Befestigung eines medizinischen Instruments 2 an einer Aufnahmevorrichtung 4. Medizinisches Instrument 2 und Aufnahmevorrichtung 4 werden hierbei derart miteinander gekoppelt, dass diese sicher miteinander verbunden sind, ohne sich im Betrieb zu lösen, und an ihrer Kontaktfläche fluiddicht miteinander verbunden sind.

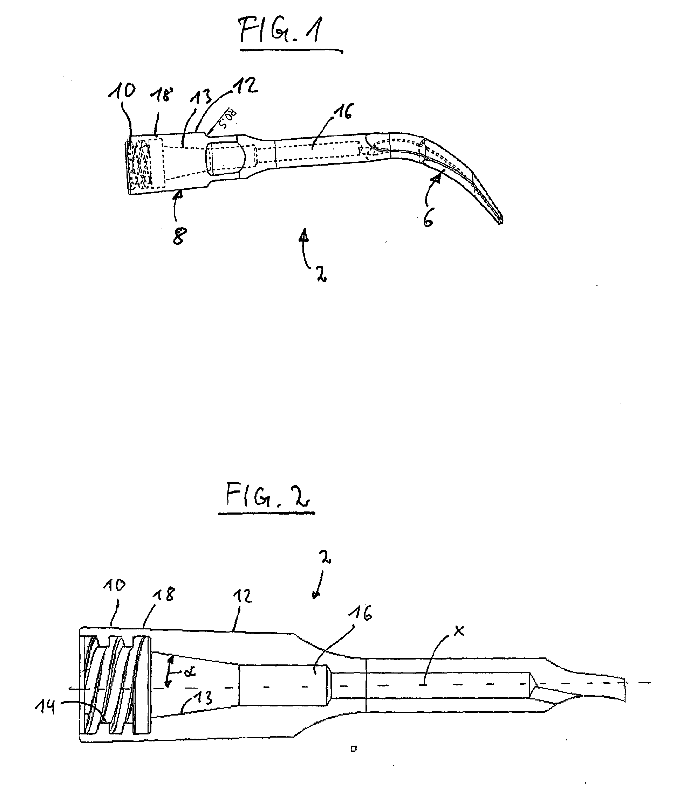

In den Figs. 1 und 2 ist eine beispielhafte Ausführungsform des erfindungsgemäßen medizinischen Instruments 2 abgebildet. Das medizinische Instrument 2 weist einen Behandlungsbereich 6 sowie einen Montagebereich 8 auf. Der Behandlungsbereich 6 befindet sich im Wesentlich an einem distalen Ende bzw. einem Endbereich des medizinischen Instruments 2 und dient der Behandlung eines Patienten. Der Behandlungsbereich 6 kann insbesondere eine gekrümmte, spitze Konfiguration aufweisen.

Der Montagebereich 8 ist vorgesehen, um das medizinische Instrument 2 an der Aufnahmeeinrichtung 4 zu befestigen bzw. zu montieren. Hierfür weist der Montagebereich 8 einen Befestigungsbereich 10 sowie einen Kontaktbereich 12 auf.

Der Befestigungsbereich 10 ist einem dem Behandlungsbereich 6 im Wesentlich entgegengesetzten Ende des medizinischen Instruments 2 ausgebildet und weist Eingriffsmittel 14 zum Eingriff mit entsprechenden Mitteln der Aufnahmevorrichtung 4 auf. Das medizinische Instrument 2 ist als Hohlkörper ausgebildet und weist einen Kanal 16 zur Durchführung eines Fluids, beispielsweise einer Spüllösung, entlang einer Längsachse X auf. Die Eingriffsmittel 14 sind hierbei an einem distalen Ende des medizinischen Instruments 2 an der inneren Mantelfläche des medizinischen Instruments 2 vorgesehen. Die Eingriffsmittel 14 sind hierbei als Gewinde ausgebildet, insbesondere in Form eines Trapezgewindes. Das Trapezgewinde weist zweckmäßigerweise aufgrund seiner geraden Flanken eine große Auflagefläche auf und kann - im Vergleich zu einem normalen Spitzgewinde - erheblich höhere Kräfte aufnehmen. Die Flankenwinkel des Trapezgewindes liegen zweckmäßigerweise in einem Bereich zwischen etwa 16° und etwa 85°, vorzugsweise zwischen etwa 70° und etwa 80° und besonders bevorzugterweise etwa 75°.

Das Schraubgewinde weist in der dargestellten Ausführungsform zwei Gewindegänge auf, wodurch die Montage des medizinischen Instruments 2 an der Aufnahmevorrichtung 4 vereinfacht werden kann, wobei aufgrund des Trapezgewindes eine ausreichende Montagesicherheit vorgesehen ist.

Der Kontaktbereich 12 ist an der inneren Mantelfläche des als Hohlkörper ausgebildeten medizinischen Instruments 2 vorgesehen. Wie ersichtlich, ist der Kontaktbereich 12 als sich verjüngender Teil ausgebildet, wobei sich der Kontaktbereich 12 in Richtung des Behandlungsbereichs 6 des medizinischen Instruments 2 verjüngt. In anderen Worten erweitert sich der Kontaktbereich 12 in Richtung des Befestigungsbereichs 10. Der Kontaktbereich 12 bzw. der sich verjüngende Teil hiervon weist zweckmäßigerweise einen Winkel bzw. Konuswinkel α zu der Längsachse X des medizinischen Instruments 2 von ca. 8° auf. Es hat sich gezeigt, dass Winkel von etwa 2° bis etwa 35°, vorzugsweise etwa 3° bis etwa 20°, und besonders bevorzugterweise etwa 4° bis etwa 10° besonders vorteilhaft sind. Dies ist der Fall, da der Konuswinkel α die Steifigkeit der Befestigung des medizinischen Instruments 2 an der Aufnahmeeinrichtung 4 mitbestimmt.

Der Kontaktbereich 12 ist ausgelegt, mit einem im Wesentlichen hierzu formkongruenten Teil der Aufnahmevorrichtung zu kontaktieren, um das medizinische Instrument 2 gegenüber der Aufnahmevorrichtung 4 in deren Verbindungsbereich gegeneinander abzudichten.

Zwischen Befestigungsbereich 10 und Kontaktbereich 12 weist das medizinische Instrument 2 zweckmäßigerweise einen Deformationsbereich 18 auf, der bei einem an die Aufnahmevorrichtung 4 montierten medizinischen Instrument 2 in Dehnungsspannung versetzt wird.

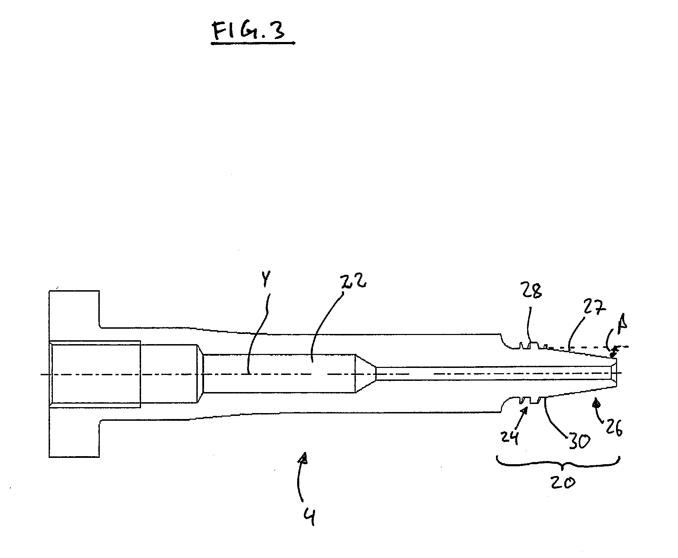

In Fig. 3 ist eine bevorzugte Ausführungsform der erfindungsgemäßen Aufnahmevorrichtung dargestellt. Die Aufnahmevorrichtung 4 weist einen Montageabschnitt 20 auf, über welchen das medizinische Instrument 2 an der Aufnahmevorrichtung 4 befestigt, bzw. montiert bzw. angeordnet bzw. festgelegt wird. Entsprechend dem medizinischen Instrument 2 ist auch die Aufnahmevorrichtung 4 als Hohlkörper ausgebildet, wobei ein Kanal 22 entlang einer Längsachse Y vorgesehen ist, welcher so mit dem Kanal 16 in Fluidverbindung steht, dass ein dem Kanal 22 zugeführtes Fluid in den Kanal 16 des medizinischen Instruments geleitet wird und an dessen Behandlungsbereich 6 herausgeführt wird.

Zur Befestigung des medizinischen Instruments 2 an der Aufnahmevorrichtung 4 weist die Aufnahmevorrichtung 4 im Bereich des Montageabschnitts 20 einen Befestigungsabschnitt 24 sowie einen Kontaktabschnitt 26 auf.

Der Befestigungsabschnitt 24 umfasst Eingriffsmittel 28, die mit den Eingriffsmitteln 14 des medizinischen Instruments 2 in Eingriff gebracht werden können. Die Eingriffsmittel 28 sind zweckmäßigerweise als Schraubgewinde ausgebildet, wobei das Schraubgewinde bevorzugterweise in Form eines Trapezgewindes mit zwei Gewindegängen vorgesehen ist. Hierdurch ist es möglich, eine einfache Montage der beiden Elemente bei hoher Befestigungssicherheit zu ermöglichen. Der Kontaktabschnitt 26 weist einen sich verjüngenden Teil 27 auf, der eine konusförmige Konfiguration besitzt. Der Konuswinkel β der Mantelfläche des sich verjüngenden Teils 27 in Bezug auf die Längsachse Y der Aufnahmevorrichtung 4 entspricht im Wesentlichen dem Konuswinkel α des medizinischen Instruments 2. Hierdurch wird gewährleistet, dass ein im Wesentlichen flächiger Kontakt des Kontaktbereichs 12 und des Kontaktabschnitts 26 gegeben ist, wodurch eine besonders hohe Dichtigkeit der Verbindung gewährleistet wird. Der sich verjüngende Teil 27 verjüngt sich hierbei zu dem distalen Ende der Aufnahmevorrichtung 4 hin, welches mit dem medizinischen Instrument 2 verbunden wird.

Zwischen dem Befestigungsabschnitt 24 und Kontaktabschnitt 26 weist der Montageabschnitt 20 weiterhin einen Deformationsabschnitt 30 auf, der bei einem an der Aufnahmevorrichtung 4 montierten medizinischen Instrument 2 unter parallel zur Längsachse Y gerichteter Druckspannung versetzt wird.

Das medizinische Instrument 2 und die Aufnahmevorrichtung 4 können aus beliebigen, auch unterschiedlichen Materialien hergestellt sein. Besonders vorteilhafterweise ist das medizinische Instrument 2 aus Stahl ausgebildet, wohingegen die Aufnahmevorrichtung 4 aus Titan geschaffen ist.

Um eine während der Nutzung der Elemente zuverlässige Abdichtung und Befestigung zu gewährleisten, ist der quer bzw. im Wesentlichen senkrecht zur Längsachse X gewählte Querschnitt des medizinischen Instruments 2 im Bereich des Deformationsbereichs 18 und der quer bzw. im Wesentlichen senkrecht zur Längsachse Y stehende Querschnitt der Aufnahmevorrichtung 4 im Bereich des Deformationsabschnitts 30 derart gewählt, dass das Produkt aus der Querschnittsfläche des Deformationsbereichs 18 und das Elastizitätsmodul des Materials des medizinischen Instruments 2 etwa 0,8 bis etwa 1,2, vorzugsweise etwa 0,9 bis etwa 1,1 und besonders vorzugsweise etwa gleich dem Produkt aus der im Wesentlichen senkrecht zur Längsachse Y stehenden Querschnittsfläche des Deformationsabschnitt 30 und dem Elastizitätsmodul des Materials der Aufnahmevorrichtung 4 ist.

### Bezugszeichenliste

- 2: Medizinisches Instrument
- 4: Aufnahmevorrichtung
- 6: Behandlungsbereich
- 8: Montagebereich
- 10: Befestigungsbereich
- 12: Kontaktbereich
- 13: sich verjüngender Teil
- 14: Eingriffsmittel
- 16: Kanal
- 18: Deformationsbereich
- 20: Montageabschnitt
- 22: Kanal
- 24: Befestigungsabschnitt
- 26: Kontaktabschnitt
- 27: sich verjüngender Teil
- 28: Eingriffsmittel
- 30: Deformationsabschnitt
- α: Konuswinkel
- β: Konuswinkel
- X: Längsachse
- Y: Längsachse

## Patentansprüche

1. Befestigungssystem zur Befestigung eines medizinischen Instruments (2) an einer Aufnahmevorrichtung (4), wobei das medizinische Instrument derart mit der Aufnahmevorrichtung koppelbar ist, dass ein sich verjüngender Teil (13, 27) der Aufnahmevorrichtung (4) oder des medizinischen Instruments (2) mit einem im Wesentlichen formkongruenten Teil (13, 27) des verbleibenden medizinischen Instruments (2) oder der Aufnahmevorrichtung (4) in Eingriff bringbar ist.

2. Befestigungssystem nach Anspruch 1, wobei ein Kontaktbereich (12) an dem medizinischen Instrument (2) und ein hiermit in Kontakt bringbarer Kontaktabschnitt (26) an der Aufnahmevorrichtung (4) vorgesehen sind.

3. Befestigungssystem nach Anspruch 2, wobei der sich verjüngende Teil (13, 27) an dem Kontaktbereich (12) oder dem Kontaktabschnitt (26) ausgebildet ist.

4. Befestigungssystem nach einem der vorhergehenden Ansprüche, wobei der sich verjüngende Teil (13, 27) eine konusförmige Konfiguration besitzt.

5. Befestigungssystem nach einem der vorhergehenden Ansprüche, wobei das medizinische Instrument (2) und die Aufnahmevorrichtung (4) als Hohlkörper zur Durchführung eines Fluids ausgebildet sind.

6. Befestigungssystem nach einem der vorhergehenden Ansprüche, wobei ein Befestigungsbereich (10)an dem medizinischen Instrument (2) und ein hiermit in Eingriff bringbarer Befestigungsabschnitt (24) an der Aufnahmevorrichtung (4)vorgesehen sind.

7. Befestigungssystem nach Anspruch 6, wobei der Befestigungsbereich (10) und der Befestigungsabschnitt (24) jeweils Eingriffsmittel (14, 28) zum gegenseitigen Eingriff aufweisen.

8. Befestigungssystem nach Anspruch 7, wobei die Eingriffsmittel (28) des Befestigungsabschnitts (24) außenliegend angeordnet sind.

9. Befestigungssystem nach einem der Ansprüche 7 oder 8, wobei die Eingriffsmittel (14) des Befestigungsbereichs (10) an einer innenliegenden Mantelfläche des als Hohlkörper ausgebildeten medizinischen Instruments (2) ausgebildet sind.

10. Befestigungssystem nach einem der Ansprüche 7 - 9, wobei die Eingriffsmittel (14, 28) als Schraubgewinde in Form eines Trapezgewindes ausgebildet sind.

11. Befestigungssystem nach Anspruch 10, wobei das Schraubgewinde weniger als sechs, vorzugsweise weniger als vier und besonders bevorzugterweise zwei Gewindegänge aufweist.

12. Befestigungssystem nach einem der vorhergehenden Ansprüche, wobei das medizinische Instrument (2) zwischen Befestigungsbereich (10) und Kontaktbereich (12) einen Deformationsbereich (18) aufweist.

13. Befestigungssystem nach einem der vorhergehenden Ansprüche, wobei die Aufnahmevorrichtung zwischen Befestigungsabschnitt (24) und Kontaktabschnitt (26) einen Deformationsabschnitt (30) aufweist.

14. Befestigungssystem nach den Ansprüchen 12 und 13, wobei das Produkt aus Querschnittsfläche des Deformationsbereichs (18) und Elastizitätsmodul des Materials des medizinischen Instruments (2) zwischen etwa 0,7 bis etwa 1,4, vorzugsweise etwa 0,85 bis etwa 1,2 und besonders bevorzugt etwa 0,95 bis etwa 1,05 mal dem Produkt aus Querschnittsfläche des Deformationsabschnitts (30) und Elastizitätsmodul des Materials der Aufnahmevorrichtung (4) ist.

15. Medizinisches Instrument, insbesondere zahnärztliches Instrument zur Beseitigung von Zahnstein oder zur Behandlung von Parodontose, umfassend einen Behandlungsbereich (6) und einem Montagebereich (8) zur Befestigung an einer Aufnahmevorrichtung (4), wobei der Montagebereich einen sich verjüngenden Teil (13) aufweist, um mit einem im Wesentlichen formkongruenten Teil (27) der Aufnahmevorrichtung (4) in Eingriff gebracht zu werden.

16. Medizinisches Instrument nach Anspruch 15, wobei ein Kontaktbereich (12) an dem medizinischen Instrument (2)vorgesehen ist, an welchem der sich verjüngende Teil (13) ausgebildet ist.

17. Medizinisches Instrument nach einem der Ansprüche 15 - 16, wobei der sich verjüngende Teil (13) eine konusförmige Konfiguration besitzt.

18. Medizinisches Instrument nach einem der Ansprüche 15 - 17, welches als Hohlkörper zur Durchführung eines Fluids ausgebildet ist.

19. Medizinisches Instrument nach einem der Ansprüche 15 - 18, wobei ein Befestigungsbereich (10) mit Eingriffsmitteln (14) zur Schaffung eines Eingriffs mit der Aufnahmevorrichtung (4) vorgesehen ist.

20. Medizinisches Instrument nach Anspruch 19, wobei die Eingriffsmittel (14) des Befestigungsbereichs (10) an einer innen liegenden Mantelfläche des als Hohlkörper ausgebildeten medizinischen Instruments (2) ausgebildet sind.

21. Medizinisches Instrument nach einem der Ansprüche 19 oder 20, wobei die Eingriffsmittel (14) als Schraubgewinde in Form eines Trapezgewindes ausgebildet sind.

22. Medizinisches Instrument nach Anspruch 21, wobei das Schraubgewinde weniger als sechs, vorzugsweise weniger als vier und besonders bevorzugterweise zwei Gewindegänge aufweist.

23. Medizinisches Instrument nach einem der Ansprüche 15 - 22, wobei zwischen Befestigungsbereich (10) und Kontaktbereich (12) ein Deformationsbereich (18) vorgesehen ist.

24. Aufnahmevorrichtung (4) für ein medizinisches Instrument (2), insbesondere ein Handstück für ein zahnärztliches Instrument zur Beseitigung von Zahnstein und zur Behandlung von Parodontose, umfassend einen Montageabschnitt (20) mit einem sich verjüngenden Teil (27), um mit einem im Wesentlichen formkongruenten Teil (13) des medizinischen Instruments (2) in Eingriff gebracht zu werden.
